# EUROPEAN PATENT APPLICATION

(11) **EP 1 065 171 A1**
(43) Date of publication of application: **03.01.2001**
(21) Application number: 00112592.1
(22) Date of filing: 14.06.2000
(51) Int. Cl.: C02F 1/68, C02F 1/48, C02F 1/30, A61K 7/00, C02F 103/02

(54) **Water with high polarity**

(30) Priority: 17.06.1999 JP 17163799
(71) Applicant: Hattori, Toshimitsu, Fukuoka-shi, Fukuoka (JP)
(72) Inventor: Hattori, Toshimitsu, Fukuoka-shi, Fukuoka (JP); Matsushita, Kazuhiro, Akishima-shi, Tokyo 196-0025 (JP)
(74) Representative: Bühling, Gerhard, Dipl.-Chem.

(57) **Abstract**

The present invention provides water having the following characteristics. When heavy water is added to the water of the present invention so that the final concentration of the heavy water is 10% and the resultant mixture is subjected to ¹H-NMR measurement at a frequency of 400MHz, at a resolving power of ±0.01Hz and at 22°C, the obtained peak is positioned at a frequency that is at least 0.5Hz higher than the frequency of the peak of water obtained by subjecting pure water to a magnetization treatment, the peak being observed in ¹H-NMR measurement under the same conditions.

## Description

### BACKGROUND OF THE INVENTION

### 1. Field of the Invention

The present invention relates to water, more specifically, novel water with high polarity.

### 2. Description of the Related Art

Conventionally, there have been attempts to treat water so as to reduce the size of a water molecular cluster. For example, a method is known in which a magnetization treatment and a far infrared radiation treatment are used in combination in order to make tap water suitable for drinking. For example, Japanese Laid-Open Patent Publication Nos. 2-122888, 4-222698, 6-170375, 6-218381, 7-132286, 5-115898, 5-293491, 2-211288 and 7-51679 describe that a magnetization treatment and a far infrared radiation treatment are used in combination for the purpose of sterilizing and purifying tap water. Japanese Laid-Open Patent Publication No. 5-269468 discloses a method of activating water by the magnetization treatment, the far infrared radiation treatment and the bubbling of oxygen or carbon dioxide therein.

However, in any of these treatments, tap water or water containing minerals is treated so as to be suitable for drinking. When the water treated with these procedures is used for cosmetics such as skin lotion and the like, the permeability through the skin may be enhanced, but it is insufficient for use in the field of cosmetics.

Therefore, water that has excellent permeability through the skin and moisture-retention properties and exerts excellent effects when used in the field of cosmetics, e.g. used for skin lotion is more desirable.

### SUMMARY OF THE INVENTION

The present invention includes novel water with high polarity. The water is characterized as follows. When heavy water is added to the water of the present invention so that the final concentration of the heavy water is 10% and the resultant mixture is subjected to ¹H-NMR measurement at a frequency of 400MHz, at a resolving power of ±0.01Hz and at 22°C, the obtained peak is positioned at a frequency that is at least 0.5Hz higher than the frequency of the peak of water obtained by subjecting pure water to a magnetization treatment, the peak being observed in ¹H-NMR measurement under the same conditions.

In a preferred embodiment, the water is obtained by subjecting purified water to a magnetization treatment and a contactless electromagnetic treatment.

In a preferred embodiment, the water is obtained by subjecting pure water to a magnetization treatment and a contactless electromagnetic treatment.

In a preferred embodiment, the contactless electromagnetic treatment is a far infrared radiation treatment.

The present invention also includes a cosmetic product comprising the above-mentioned water.

Thus, the present invention achieves the following objectives of, providing novel water that has high polarity and excellent permeability through the skin and moisture-retention properties, and providing water having the above characteristics that can be used for cosmetic products such as skin lotion, shampoo, hair rinse, hair treatment, lotion, pack, water foundation or the like and exerts excellent effects.

These and other advantages of the present invention will become apparent to those skilled in the art upon reading and understanding the following detailed description with reference to the accompanying figures.

### BRIEF DESCRIPTION OF THE DRAWINGS

Fig. 1 is a ¹H-NMR chart of the treated water samples obtained in Examples.
Fig. 2 is a graph showing the amount of residual moisture in the epidermal stratum corneum in experiments conducted with the treated water obtained in Examples.
Fig. 3 is a graph showing significant differences in the amount of residual moisture in the epidermal stratum corneum of treated water (a) and treated water (c) obtained in Examples. In this graph, * indicates P < 0.05, and ** indicates P < 0.01 in the significant difference test.
Fig. 4 is a graph showing significant differences in the amount of residual moisture in the epidermal stratum corneum of treated water (b) and treated water (c) obtained in Examples. In this graph, * indicates P < 0.05 in the significant difference test.
Fig. 5 is a graph showing significant differences in the amount of residual moisture in the epidermal stratum corneum of treated water (a) and treated water (d) obtained in Examples. In this graph, * indicates P < 0.05, and ** indicates P < 0.01 in the significant difference test.
Fig. 6 is a graph showing significant differences in the amount of residual moisture in the epidermal stratum corneum of treated water (b) and treated water (d) obtained in Examples. In this graph, ** indicates P < 0.01 in the significant difference test.

### DESCRIPTION OF THE PREFERRED EMBODIMENT

The water of the present invention has the following feature. When heavy water is added to the water so that the final concentration of the heavy water is 10% and the resultant mixture is subjected to ¹H-NMR measurement at a frequency of 400MHz, at a resolving power of ±0.01Hz and at 22°C, the obtained peak is positioned at a frequency that is at least 0.5Hz higher than the frequency of the peak of water obtained by subjecting pure water to a magnetization treatment, the peak being observed in ¹H-NMR measurement under the same conditions. Water having such nature is unknown at present.

The water of the present invention is obtained by, for example subjecting purified water or pure water to a magnetization treatment and a contactless electromagnetic treatment.

In this specification, pure water refers to substantially pure water that is obtained by demineralizing tap water or the like and has a resistivity of about 1MΩ · cm or more, preferably 11 to 18.2MΩ · cm, and includes extrapure water.

In this specification, purified water means purified water designated in the Japanese pharmacopeia and refers to water obtained by distilling qua communis under the Japanese pharmacopeia (i.e., tap water or well water) or passing it through an ion exchange resin for purification.

The magnetization treatment of water means applying magnetic force to water, and for example, an apparatus described in USP5,584,994 can be used as a magnetization treatment apparatus. For example, a preferable apparatus to use is as follows. Water is circulated and a magnet device is provided in a predetermined position in the circulating water path. The magnet device applies magnetic force to the water substantially perpendicularly to the water stream. As the magnet device, a device where a plurality of magnets are arranged in parallel or in series in the circulating water path also can be used to achieve the same results.

In general, the magnetization treatment using the above-described device is performed by applying magnetic force of 0.07T to 15T (tesla: 1 tesla equals to 10,000gauss) to the water for a suitable period of time. Preferably, the magnetic force is 0.3T to 3T, and more preferably 0.8T to 2T, for example, 1.2T.

The contactless electromagnetic treatment includes a far infrared radiation treatment and an electric field treatment. The far infrared radiation treatment is performed by, for example providing a ceramic that can emit far infrared rays with a wavelength of 4 to 24µm in the circulating water path of the above-described apparatus (for example, by covering a tubular circulating water path with a ceramic cylindrical jacket) and irradiating water with far infrared rays to achieve magnetization of the water.

The emissivity of the far infrared radiation is preferably 80% or more, and more preferably 90% or more

The electric field treatment can be performed by applying a voltage of 50 to 8000V, preferably 3000 to 5000V to the circulating water path.

The magnetization treatment and the contactless electromagnetic treatment can be performed at the same time. Alternatively, the contactless electromagnetic treatment can be performed after the magnetization treatment, or the magnetization treatment can be performed after the contactless electromagnetic treatment.

A peak obtained by ¹H-NMR measurement of the thus obtained water is positioned at a frequency that is at least 0.5Hz higher than the frequency of the peak of water obtained by subjecting pure water to the magnetization treatment. The ¹H-NMR measurement is conducted under the conditions that heavy water is added to the above-mentioned water so that the final concentration of heavy water is 10%, and the resultant mixture is subjected to ¹H-NMR measurement at a frequency of 400MHz, at a resolving power of ±0.01Hz, and at 22°C. The peak observed in ¹H-NMR measurement generally is positioned at a frequency that is 0.5 to 3.0Hz, preferably 0.8 to 3.0Hz, and more preferably 1.0 to 3.0Hz higher than the frequency of the peak of water obtained by subjecting pure water to the magnetization treatment. It has been indicated that such water has high polarity and a novel nature. For example, as shown in the following examples, water obtained by subjecting purified water to the magnetization treatment and the far infrared radiation treatment and water obtained by subjecting pure water to the magnetization treatment and the far infrared radiation treatment have peaks that are 1.14Hz and 1.42Hz, respectively, higher than the frequency of the peak of water obtained by subjecting pure water only to the magnetization treatment in ¹H-NMR measurements. These peaks are observed when heavy water is added to a sample water so that the final concentration of heavy water is 10%, and the resultant mixture is subjected to ¹H-NMR measurement at a frequency of 400MHz, at a resolving power of ±0.01Hz, and at 22°C. The novel water of the present invention has higher permeability through the skin and moisture-retention properties than those of conventional water, as shown in the examples described below.

The water of the present invention can be used in all the fields using water as an ingredient. For example, in the field of cosmetic products, the water can be used in cosmetic cream, skin lotion, shampoo, hair rinse, hair treatment, hair liquid, lotion, pack, water foundation or any other cosmetic products using water. The water of the present invention can be used also as water in the production of powdery cosmetic products.

### Examples

### Example 1

### (Production of water of the present invention)

An apparatus for treating water obtained by modifying the apparatus for producing magnetized water described in USP5,584,994 was prepared. In this apparatus for treating water, water discharged from a water tank circulates through a circulating pipe and returns to the tank. An apparatus for applying magnetism is provided in a predetermined position in the water path of the circulating pipe. In this portion, water passes through slit-shaped water channels with an interval of 2mm, and magnetic force is applied from the magnet device to the water. Furthermore, a ceramic piece is provided in a portion of the circulating pipe, and circulated water is irradiated with far infrared rays. In this apparatus, purified water designated in the Japanese pharmacopeia or pure water was circulated at a flow rate of about 15m/min. Then, a magnetization treatment was performed with a magnetic force of 1.2T, and then, a far infrared radiation treatment was also performed. The water was circulated for 24 hours, and thus a treated water was obtained. The treated water obtained by treating purified water is referred to as treated water (c). The treated water obtained by treating pure water is referred to as treated water (d). For comparison, water obtained by treating tap water is referred to as treated water (a), and water obtained by subjecting pure water to a magnetization treatment with the same apparatus without the ceramic is referred to as treated water (b). The treated water (b) was used as a standard.

The half band width of the ¹⁷O-NMR of each water was measured at 20°C, a frequency 32MHz and a cumulative number of times of 2048 by JNM EX-270 FT-NMR spectrometer manufactured by Nippon Electronics Co., Ltd. The half band widths of treated waters (a), (b), (c), and (d) were 130Hz, 74Hz, 78Hz, and 75Hz, respectively.

### (Measurement of ¹H-NMR)

Heavy water was added to the water samples obtained as above so that 10% heavy water solutions were prepared. Heavy water was added to stabilize the magnetic field and to control the magnetic field to be uniform by the resonance of the deuterium nucleus.

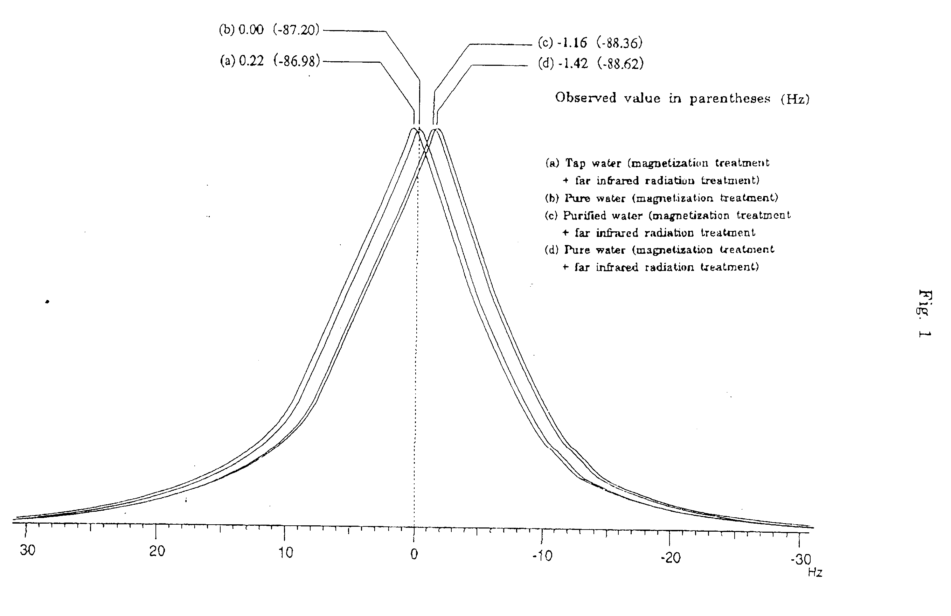

The measurement of ¹H-NMR was performed at a frequency of 400MHz, a controlled temperature of 22°C±0.1°C, a cumulative number of times of 4 and a resolving power of 0.01Hz by JNM LA-400 FT-NMR spectrometer manufactured by Nippon Electronics Co., Ltd. The measurement of ¹H-NMR was performed 10 times for each sample. Table 1 shows the peak frequencies in each chart. Fig. 1 shows the chart of ¹H-NMR spectrum of the treated waters (a), (b), (c) and (d) (the peak of the treated water (b) is used as the standard (0Hz)).

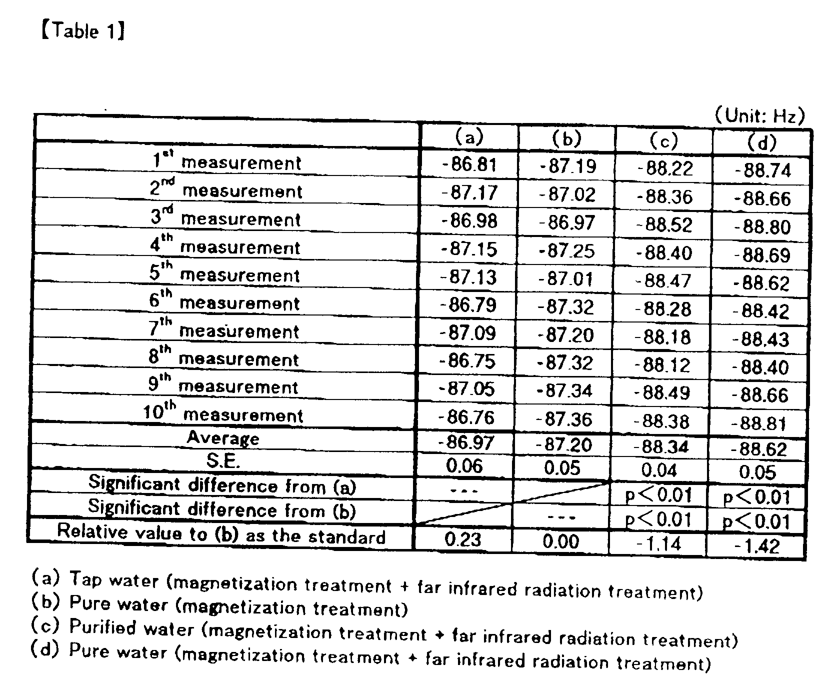

Referring to Fig. 1 and Table 1, when the treated water (b) is the standard, the peak of the treated water (a) in ¹H-NMR chart is 0.23Hz shifted to lower frequency. The peaks of the treated waters (c) and (d) in ¹H-NMR chart are 1.14Hz and 1.42Hz shifted to higher frequency, respectively.

Then, t-test with a significance level of less than 1% was performed between the treated waters (c) and (a), between the treated waters (c) and (b), between the treated waters (d) and (a), and between the treated waters (d) and (b). Significant differences were observed in all the tests.

Furthermore, ¹H-NMR measurement was performed with respect to tap water, well water, thawed water, spring water and purified water that are not subjected to any treatment in the same manner as above. The peaks thereof were 0.36Hz, 0.38Hz, 0.38Hz, 0.39Hz and 0.33Hz shifted to lower frequency, respectively, with respect to the peak of the treated water (b) as the standard. In this way, the water of the present invention is clearly distinguished from these untreated waters Thus, it is confirmed that the water of the present invention is novel water.

### (Permeability and moisture-retention properties)

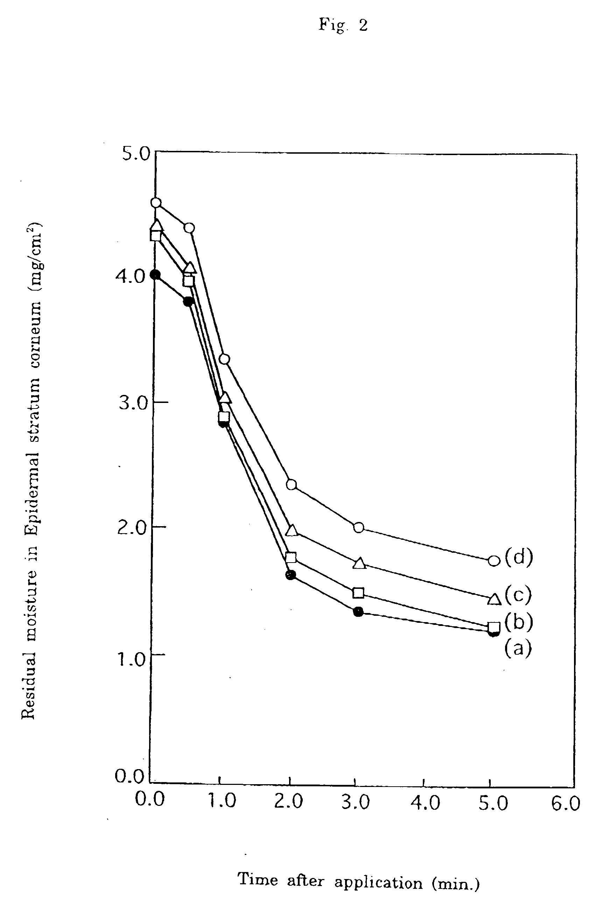

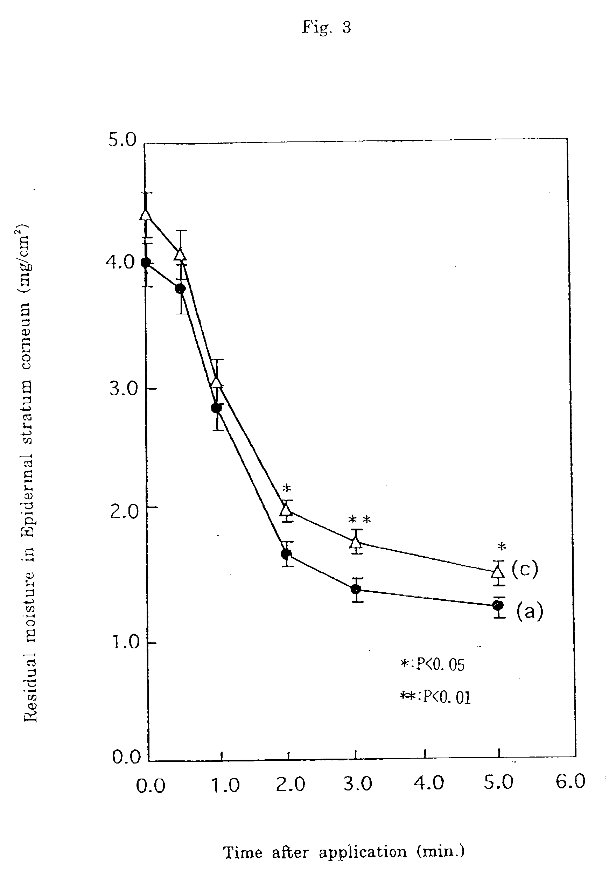

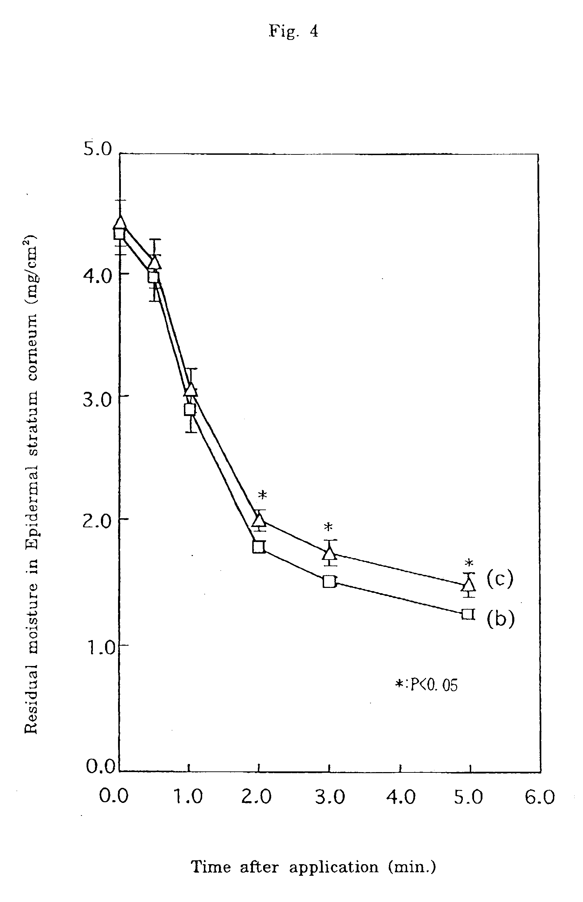

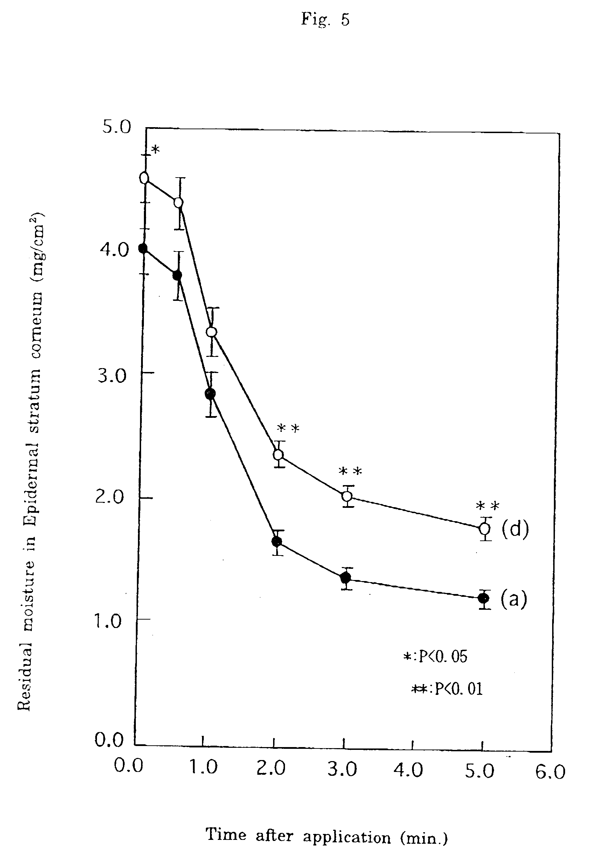

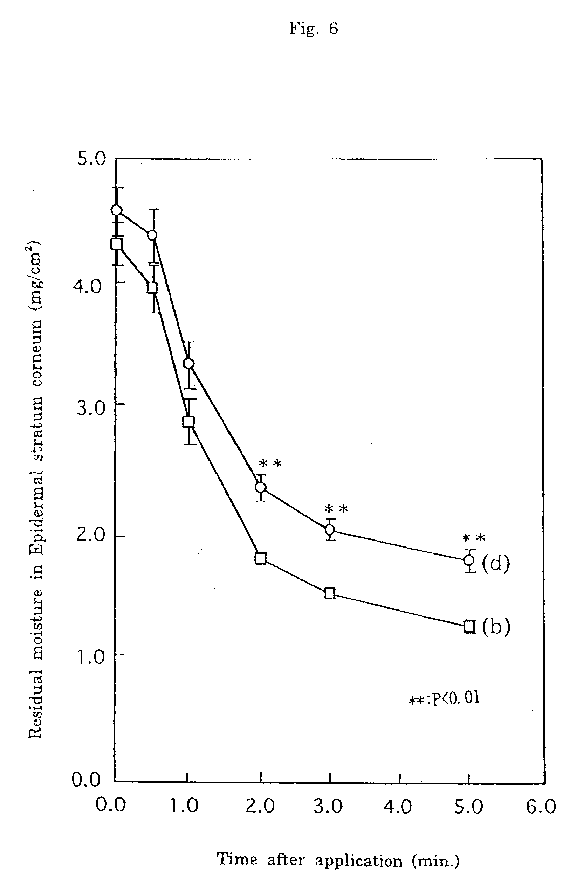

The permeability and the moisture-retention property of each of the treated waters were evaluated by the moisture content in the skin. Each treated water was applied at an application density of 34.7µl/cm² uniformly to the forearm on the flexor side of a subject, and the moisture content in the stratum corneum was measured by a device for measuring the moisture in the epidermal stratum corneum (SKICOS 401; produced by Amikku (phonetic expression) group) in a predetermined period of time later, so that the content of the residual moisture in the skin was obtained. This test was carried out to ten subjects, and the residual moisture content in the stratum corneum was obtained with respect to each of the treated waters (a), (b), (c) and (d). Fig. 2 shows the results. The treated waters (c) and (d) of the present invention had more residual moisture content at all the measurement points than those of the treated waters (a) and (b). The residual moisture content of the treated waters (c) and (d) are larger than either of the residual moisture content of the treated waters (a) and (b), especially immediately after the application and 2 minutes or more after the application. Furthermore, t-test with a significance level of less than 5% was performed with respect to the treated waters (c) and (d), relative to the treated waters (a) and (b). Significant differences were observed in almost all the measurement points. Tables 2 to 5 and Figs. 3 to 6 show the t-test results. The unit of the values in Tables 2 to 5 is mg/cm².

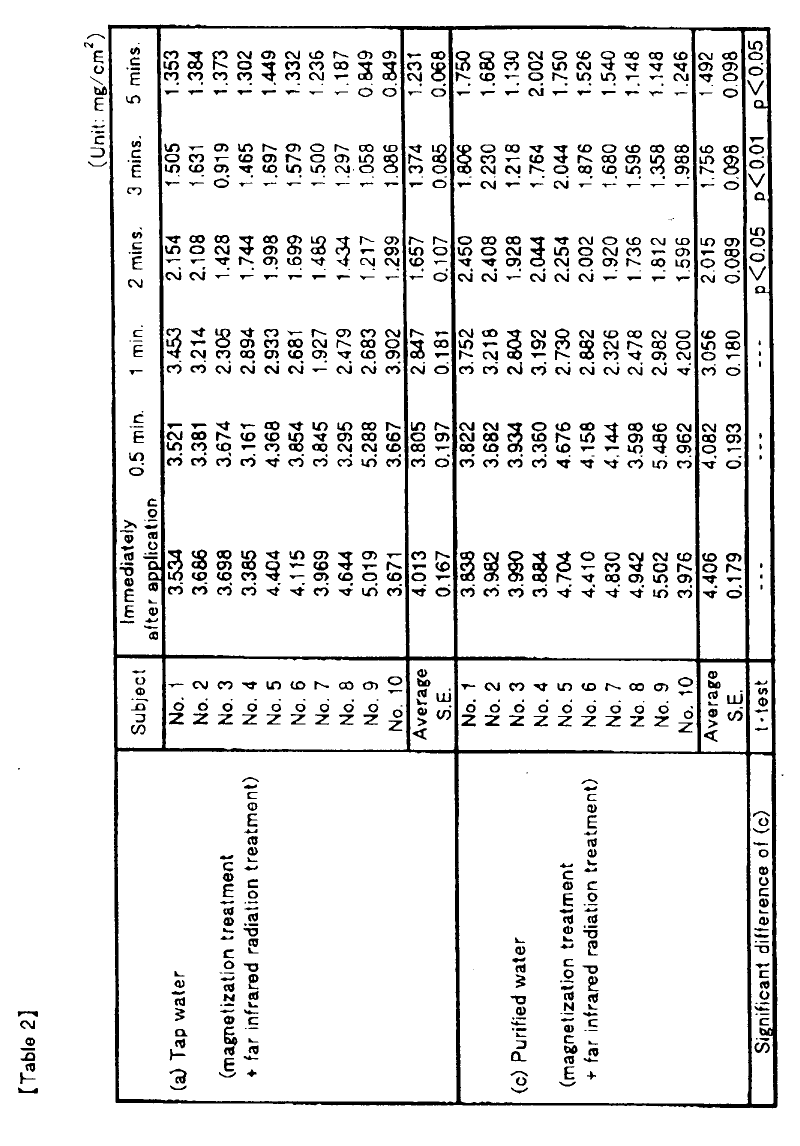

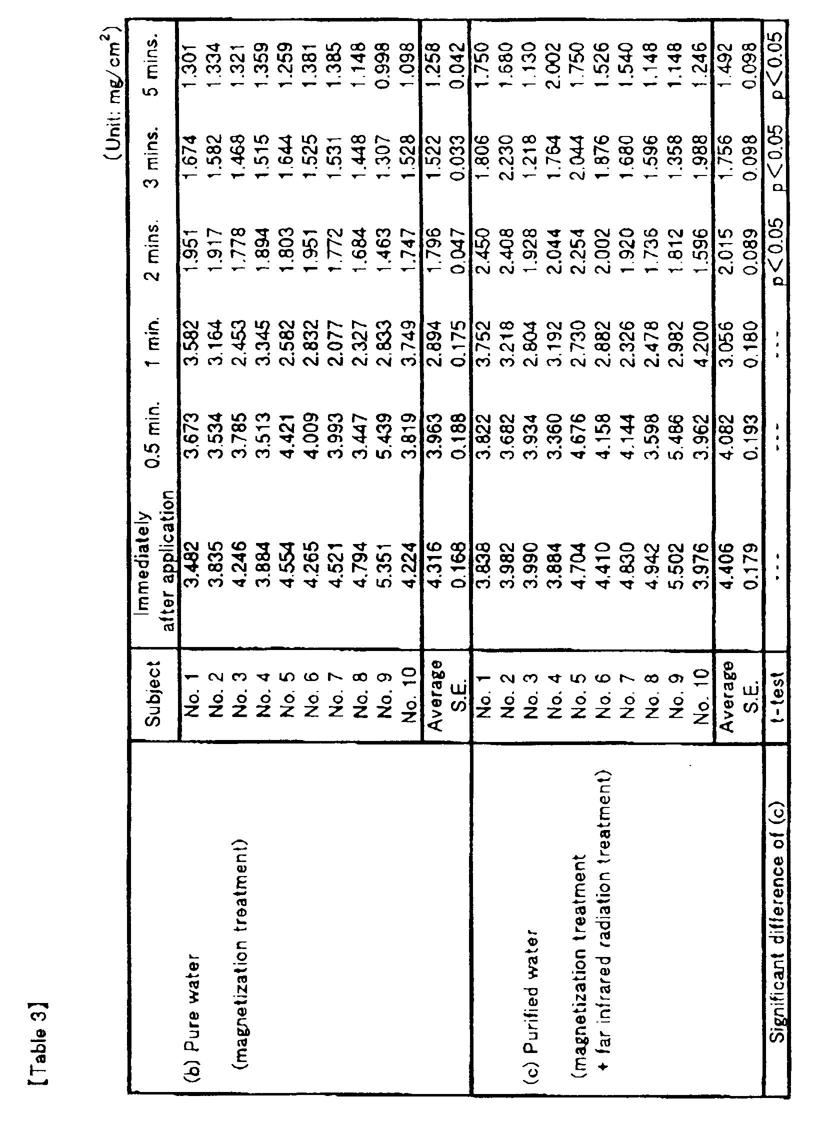

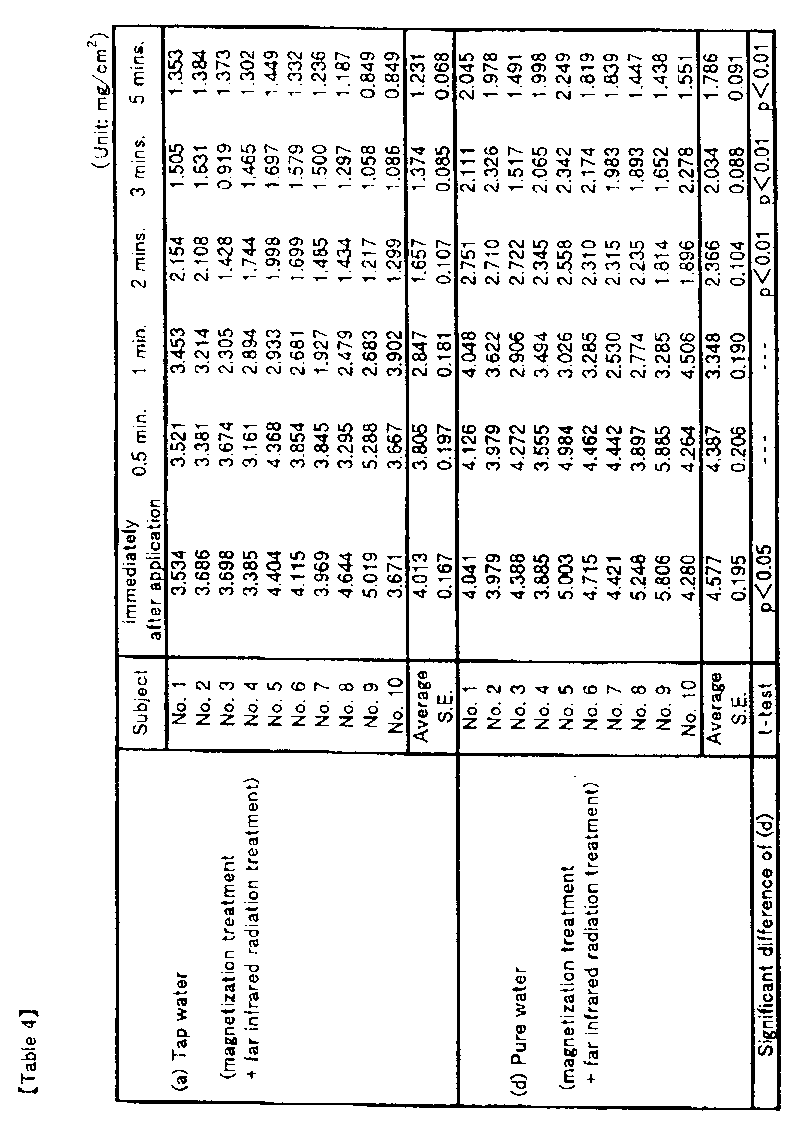

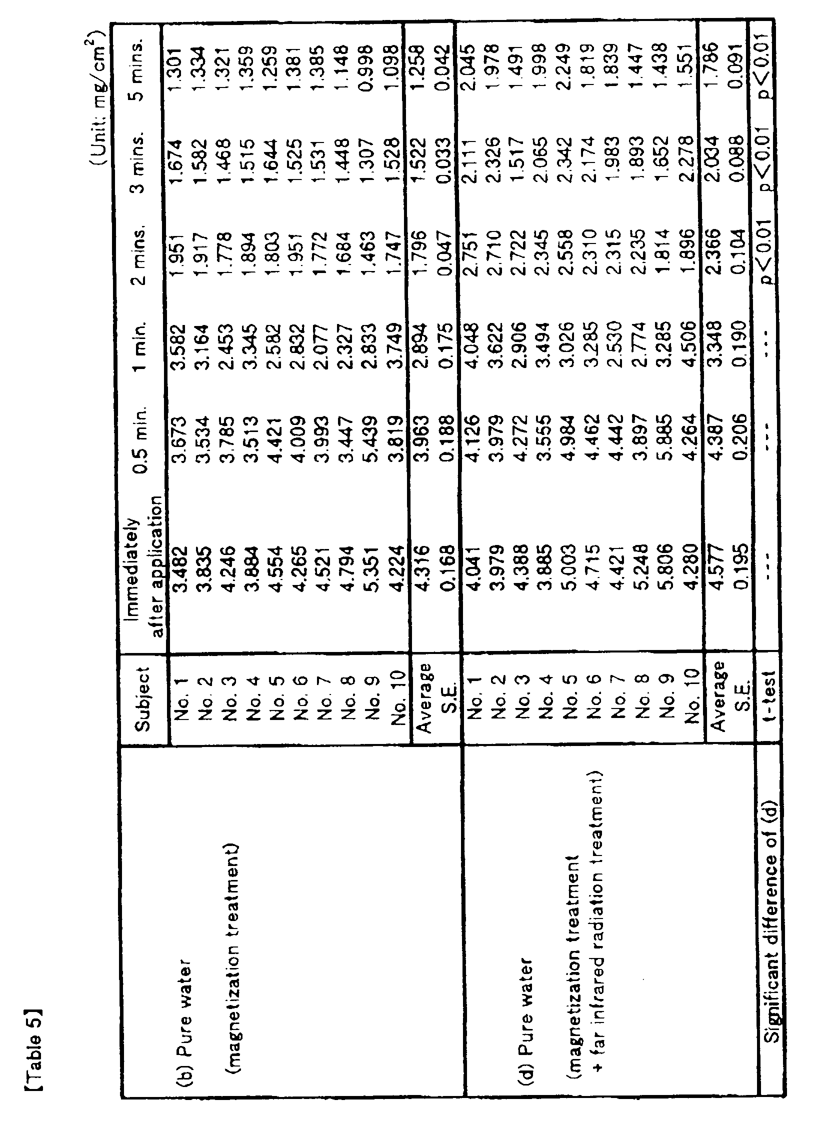

### Example 2

A cosmetic cream (cream type) having the below-indicated composition was prepared as follows:

To the treated water (c) obtained in Example 1, glycerol, propylene glycol and potassium hydroxide were added, and then mixed and stirred under heat (70°C) so as to obtain an aqueous component.

As well, stearic acid, butyl stearate and stearyl alcohol were mixed and melted by heating to obtain an oil component which was kept at 70°C. Then, perfume, an antiseptic and an antioxidant were added to this melted oil component, stirred and mixed sufficiently so as to obtain an oily solution. Then, this oily solution and the above aqueous component were mixed and stirred at 70°C to obtain a cosmetic cream.

### (Composition of the cosmetic cream)

In the composition, the percent is based on percent by weight.

| Aqueous component: | |
|---|---|
| water (treated water (c)) | 60.5% |
| alkali: potassium hydroxide | 0.5% |

| oil component: | |
|---|---|
| stearic add | 10% |
| stearyl alcohol | 4% |
| butyl stearate | 8% |

| humectant: | |
|---|---|
| propylene glycol | 10% |
| glycerol | 4% |

| emulsifier: | |
|---|---|
| glycerol monostearate | 3% |
| perfume, antiseptic, antioxidant | small amounts |
| | (total 100%) |

When the cosmetic cream was applied to the skin, the cream did not stick and was smooth and soft. The feeling of using this cream was good. Moisture-retention ability of this cosmetic cream was excellent.

### Comparative example 1

A cosmetic cream (cream type) was prepared in the same manner as that of Example 2 except that purified water was used instead of the treated water (c).

Moisture-retention ability of this cosmetic cream was inferior to that obtained in Example 2.

### Example 3

A cosmetic cream (cream type) having the below-indicated composition was prepared as follows:

To the treated water (d) obtained in Example 1, glycerol, propylene glycol and triethanolamine were added so as to obtain an aqueous component.

As well, bees wax, squalane, stearic acid and stearyl alchol were mixed to obtain an oil component which was kept at 70°C. Then, perfume, an antiseptic and an antioxidant were added to this oil component, stirred and mixed sufficiently so as to obtain an oily solution. Then, this oily solution and the above aqueous component were mixed and stirred at 70°C so as to obtain a cosmetic cream.

### (Composition of the cosmetic cream)

In the composition, the percent is based on percent by weight.

| Aqueous component: | |
|---|---|
| water (treated water (d)) | 57.5% |
| alkali: triethanolamine | 1% |

| oil component: | |
|---|---|
| bees wax | 2% |
| stearic acid | 8% |
| stearyl alchol | 5% |
| squalane | 10% |

| humectant: | |
|---|---|
| propylene glycol | 8% |
| glycerol | 4% |

| emulsifier: | |
|---|---|
| self emulsifying propylene | |
| glycol monostearate | 3% |
| polyoxyethylenecetylether | 1% |
| perfume, antiseptic, antioxidant | small amounts |
| | (total 100%) |

When the cosmetic cream was applied to the skin, the cream did not stick and was smooth and soft. The feeling of using this cream was good. Moisture-retention ability of this cosmetic cream was excellent.

### Example 4

A cosmetic cream (cream type) having the below-indicated composition was prepared as follows:

To the treated water (c) obtained in Example 1, borax was added, stirred, and mixed and retained at 70°C under heat so as to obtain an aqueous component.

As well, bees wax and liquid paraffin were mixed and melted with heat and kept at 70°C so as to obtain an oil component. Then, this melted oil component and the above aqueous component were mixed and stirred at 70°C so as to obtain a cosmetic cream.

### (Composition of the cosmetic cream)

In the composition, the percent is based on percent by weight.

| Aqueous component: | |
|---|---|
| water (treated water (c)) | 39% |
| borax | 1% |

| Oil component: | |
|---|---|
| bees wax | 10% |
| liquid paraffin | 50% |
| | (total 100%) |

When the cosmetic cream was applied to the skin, the cream did not stick and was smooth and soft. The feeling of using this cream was good. Moisture-retention ability of this cosmetic cream was excellent.

### Example 5

A cosmetic cream (milk lotion type) having the below-indicated composition was prepared as follows:

To the treated water (d) obtained in Example 1, glycerol, propylene glycol, and triethanolamine were added, stirred, and mixed, and the thus-obtained aqueous component was retained at 70°C under heat.

As well, stearic acid, cethanol, vaseline, lanolin alcohol and liquid paraffin were mixed and melted with heat and kept at 70°C so as to obtain an oil component. Then, perfume, an antiseptic and an antioxidant were added to this oil component, stirred and mixed sufficiently so as to obtain an oily solution.

Then, this oily solution and the above aqueous component were mixed and stirred at 70°C so as to obtain a cosmetic cream (milk lotion type).

### (Composition of the cosmetic cream (milk lotion type))

In the composition, the percent is based on percent by weight.

| Aqueous component: | |
|---|---|
| water (treated water (d)) | 70% |
| glycerol | 3% |
| propylene glycol | 5% |
| triethanolamine | 1% |

| Oil component: | |
|---|---|
| stearic acid | 2% |
| cethanol | 1.5% |
| vaseline | 3% |
| lanolinalcohol | 2% |
| liquid paraffin | 10% |
| perfume, antiseptic, antioxidant | small amounts |
| | (total 100%) |

When the cosmetic cream was applied to the skin, the cream did not stick and was smooth and soft. The feeling of using this cream was good. Moisture-retention ability of this cosmetic cream was excellent.

### Example 6

A cosmetic lotion having the below-indicated composition was prepared as follows:

To the treated water (c) obtained in Example 1, glycerol, propylene glycol and ultraviolet absorber were added, stirred, and mixed at an ambient temperature (25°C).

As well, an appropriate amount of softening agent (emollient), surfactant, antiseptic and perfume were added to ethanol and mixed. Then, the mixture was mixed with the above aqueous mixture so as to prepare a cosmetic lotion.

### (Composition of the cosmetic lotion)

In the composition, the percent is based on percent by weight.

| Aqueous component: | |
|---|---|
| water (treated water (c)) | 79.7% |

| humectant : | |
|---|---|
| glycerol | 6% |
| propylene glycol | 4% |

| softening agent: | |
|---|---|
| oleil alcohol | 0.1% |

| surfactant: | |
|---|---|
| polyoxyethylene lauryl ether | 1% |
| ethanol | 10% |
| perfume, antiseptic, antioxidant | small amounts |
| | (total 100%) |

When the cosmetic lotion was applied to the skin, the lotion did not stick and was soft. The feeling of using this lotion was good. Moisture-retention ability of this cosmetic lotion was excellent.

### Example 7

A foundation (cream type) having the below-indicated composition was prepared by using the treated water (d) obtained in Example 1.

### (Composition of the foundation)

In the composition the percent is based on percent by weight.

| | |
|---|---|
| water (treated water (d)) | 54.5% |
| triethanolamine | 1.2% |
| sorbitol | 2% |
| methyl p-oxybenzoate | small amount |
| pigment:(titanium oxide, talc, kaolin) and perfume | 15.5% |
| stearic acid | 5% |
| lipophilic glycerol monostearate | 2.5% |
| cetostearyl alcohol | 1% |
| propyleneglycol monolaurate | 3% |
| liquid paraffin | 7% |
| isopropylmyristate | 8% |
| butyl p-oxybenzoate | small amount |
| | (total 100%) |

When this foundation was applied to the skin, the foundation did not stick and was soft. The feeling of using this foundation was good. Moisture-retention ability of this cosmetic lotion was excellent.

### Example 8

A hair liquid having the below-indicated composition was prepared by using the treated water (c) obtained in Example 1.

### (Composition of the hair liquid)

In the composition, the percent is based on percent by weight.

| | |
|---|---|
| water (treated water (c)) | 31% |
| polyoxypropylenebutylether | 13% |
| ethanol | 56% |
| disodium edetate | small amount |
| perfume, dye | small amounts |
| | (total 100%) |

When this hair liquid was used on the hair, the hair did not stick. It felt refreshing.

### Example 9

A shampoo (transparent type) having the below-indicated composition was prepared by using the treated water (c) obtained in Example 1.

### (Composition of the shampoo)

In the composition, the percent is based on percent by weight.

| | |
|---|---|
| water (treated water (c)) | 76% |
| sodium alkylethersulfate | 16% |
| diethanolamide laurirate | 4% |
| propylene glycol | 2% |
| dye, antiseptic and ultraviolet absorber | each appropriate amount |
| | (total 100%) |

When the shampoo was used, the hair was soft. The feeling of using this shampoo was good.

### Comparative example 2

Shampoo was prepared in the same manner as that of example 9 except that purified water was used instead of the treated water (c).

When the shampoo was used, the hair was dry and loose. The feeling of use was bad.

### Example 10

A shampoo (pearl type) having the below-indicated composition was prepared by using the treated water (d) obtained in Example 1.

### (Composition of the shampoo)

In the composition, the percent is based on percent by weight.

| | |
|---|---|
| water (treated water (d)) | 76% |
| alkylsulfate triethanolamine | 15% |
| coconut fatty acid monoethanolamide | 5% |
| ethyleneglycolmonostearate | 2% |
| perfume, dye, antiseptic | appropriate amounts |
| | (total 100%) |

When the shampoo was used, the hair was soft. The feeling of using this shampoo was good.

### Example 11

A shampoo (conditioning type) having the below-indicated composition was prepared by using the treated water (c) obtained in Example 1.

### (Composition of the shampoo)

In the composition, the percent is based on percent by weight.

| | |
|---|---|
| water (treated water (c)) | 77% |
| alkylether aminesulfate | 18% |
| coconut fatty acid diethanolamide | 2% |
| cationic denatured cellulose ether | 1.5% |
| perfume, dye, antiseptic | appropriate amounts |
| | (total 100%) |

When the shampoo was used, the hair was soft. The feeling of using this shampoo was good.

### Example 12

A rinse having the below-indicated composition was prepared by using the water obtained in Example 1.

### (Composition of the rinse)

In the composition, the percent is based on percent by weight.

| | |
|---|---|
| water (treated water (c)) | 96% |
| stearyldimethylbenzylammonium chloride | 1.4% |
| stearyl alchol | 0.6% |
| glycerine monostearate | 1.5% |
| sodium chloride | 0.2% |
| perfume, dye, antiseptic | appropriate amounts |
| | (total 100%) |

When this rinse was used, the hair was soft. The feeling of using this rinse was good.

As described above, the water of the present invention has excellent permeability and moisture-retention properties.

Thus, the water of the present invention obtained by subjecting pure water to the magnetization treatment and the contactless electromagnetic treatment (preferably far infrared radiation treatment) has higher polarity and excellent permeability and moisture-retention properties. The water of the present invention can be used in all the technical fields that employ water, for example for cosmetic products such as cosmetic cream, skin lotion, shampoo, hair rinse, hair treatment, hair liquid, lotion, pack, water foundation or the like.

The present invention provides water having the following characteristics. When heavy water is added to the water of the present invention so that the final concentration of the heavy water is 10% and the resultant mixture is subjected to ¹H-NMR measurement at a frequency of 400MHz, at a resolving power of ±0.01Hz and at 22°C, the obtained peak is positioned at a frequency that is at least 0.5Hz higher than the frequency of the peak of water obtained by subjecting pure water to a magnetization treatment, the peak being observed in ¹H-NMR measurement under the same conditions.

## Claims

1. Water, wherein when heavy water is added to the water so that the final concentration of the heavy water is 10% and the resultant mixture is subjected to ¹H-NMR measurement at a frequency of 400MHz, at a resolving power of ±0.01Hz and at 22°C, the obtained peak is positioned at a frequency that is at least 0.5Hz higher than the frequency of the peak of water obtained by subjecting pure water to a magnetization treatment, the peak being observed in ¹H-NMR measurement under the same conditions.

2. The water according to claim 1, wherein the water is obtained by subjecting purified water to a magnetization treatment and a contactless electromagnetic treatment.

3. The water according to claim 1, wherein the water is obtained by subjecting pure water to a magnetization treatment and a contactless electromagnetic treatment.

4. The water according to any one of claims 1 to 3, wherein the contactless electromagnetic treatment is a far infrared radiation treatment.

5. A cosmetic product comprising the water according to any one of claims 1 to 4.
